# EUROPEAN PATENT SPECIFICATION

(11) **EP 1 600 539 B1**
(45) Date of publication and mention of the grant of the patent: **21.05.2014**
(21) Application number: 05008994.5
(22) Date of filing: 25.04.2005
(51) Int. Cl.: D03D 47/34, D03D 49/06, D03J 1/00, D03D 47/30

(54) **Display method and display device for loom**
Anzeigeverfahren und Anzeigevorrichtung für Webmaschinen
Procédé et dispositif d'affichage pour métier à tisser

(30) Priority: 26.05.2004 JP 2004156100
(43) Date of publication of application: 30.11.2005
(73) Proprietor: TSUDAKOMA KOGYO KABUSHIKI KAISHA, Kanazawa-shi, Ishikawa-ken 921-8650 (JP)
(72) Inventor: Yamazaki, Koki, Komatsu-shi, Ishikawa-ken, 923-0831 (JP); Kontani, Hideyuki, Ishikawa-ken, 929-0335 (JP); Kitamura, Emiko, Kanazawa-shi, Ishikawa-ken, 920-8216 (JP)
(74) Representative: von Samson-Himmelstjerna, Friedrich

(56) References cited:
- JP-A- 1 207 442
- JP-A- 3 161 554
- JP-A- 11 081 094

## Description

### BACKGROUND OF THE INVENTION

### 1. Field of the Invention

The present invention relates to a display method and a display device for displaying behavior of weft and warp in a loom on a screen of a display so that the operational state of the loom can be adequately evaluated.

### 2. Description of the Related Art

A technique of immediately diagnosing abnormality of a weft feeder by continuously detecting the tension of a weft yarn being inserted into a warp shed and displaying it in the form of a waveform with respect to a crank angle in a single rotation of the loom is disclosed in Japanese Unexamined Patent Application Publication No. 1-207422.

According to this publication, a sensor for detecting the weft tension is provided upstream of a main nozzle in a jet loom, and an output signal from the sensor is memorized after an analog-to-digital (A/D) conversion. Then, the weft tension is displayed as a waveform with respect to a crank angle on the basis of the memorized data. By evaluating a variation width of the waveform being displayed, an abnormal section in the weft feeder can be determined and diagnosed. The variation width is visualized by superimposing waveforms for multiple times of rotation of the loom.

According to the above-described known structure, data for multiple times of rotation of the loom is required since it is determined whether or not there is an abnormality on the basis of the variation width of the waveforms, and a large-capacity memory is required accordingly. In addition, it is not easy to compare waveforms before and after the occurrence of an abnormality on the same display screen, and thus the operability is degraded.

### SUMMARY OF THE INVENTION

In view of the above-described problems of the known structure, it is an object of the present invention to provide a display method and a display device in which an output signal from a sensor is sampled at every predetermined crank angle and data obtained in different memory periods are superimposed on a screen of a display, so that restriction by the variation in the number of rotation of the loom is completely eliminated and the operational state of the loom at different times can be easily compared with each other.

In order to attain the above-described object, according to a first aspect of the present invention, a display method for a loom having a sensor which detects the behavior of yarns, a memory which memorizes an output signal from the sensor, and a display which displays the data in the memory on a screen includes a step of storing a data set which is based on the output signal from the sensor sampled at every predetermined period and which corresponds to a predetermined memory period in response to a store command and a step of displaying waveforms corresponding to a plurality of data sets obtained in different memory periods and stored in the memory on the screen such that the waveforms are superimposed on each other in response to a display command.

In addition, the loom may sample the output signal from the sensor at every predetermined period while in operation, memorize the sampled data in the memory over the predetermined memory period, successively update the data in the memory, and store the data memorized in the memory in response to the store command.

The loom may also sample the output signal from the sensor at every predetermined period in response to the store command, successively memorize the sampled data in the memory, and store the data corresponding to the predetermined memory period in the memory.

According to a second aspect of the present invention, a display device for a loom having a sensor which detects the behavior of yarns, a memory which memorizes an output signal from the sensor, and a display which displays the data in the memory on a screen includes a sampling unit for sampling the output signal from the sensor at every predetermined period and causing the memory to memorize the sampled data set over a predetermined memory period; and a display controller which displays waveforms corresponding to a plurality of data sets obtained in different memory periods on the screen such that the waveforms are superimposed on each other in response to a display command, each of the data sets being stored in the memory in response to a store command.

The display device may further include a memory controller. In such a case, the sampling unit may sample the output signal from the sensor at every predetermined period while the loom is in operation, memorize the sampled data in the memory over the predetermined memory period, and successively update the data in the memory, and the memory controller may store the data memorized in the memory by the sampling unit and corresponding to the predetermined memory period in response to the store command.

In the display device, the sampling unit may sample the output signal from the sensor at every predetermined period in response to the store command, successively memorize the sampled data in the memory, and store the data corresponding to the predetermined memory period in the memory.

In addition, in the display device, the display controller may shift display ranges of the waveforms on the screen in response to a shift command.

In the above, the process of "successively updating the data in the memory" means that the latest data corresponding to the memory period up to the present time are memorized in time series by successively updating the latest data in the memory. In addition, the process of "storing the data memorized in the memory" means that the data memorized in the memory in the above-described manner is extracted so that the data would not be updated anymore and is rememorized as fixed data. In addition, according to the present invention, the "sensor which detects the behavior of yarns" includes, for example, a warp tension sensor, a weft tension sensor, a weft feeler, a weft release sensor used in a drum weft feeder, a dropper for detecting warp breakage, etc.

According to the present invention, the output from the sensor is sampled at every predetermined period, and the sampled data is memorized in the memory over the predetermined memory period. Then, the data stored in the memory which corresponds to the predetermined memory period is stored in response to the store command. When the store command is repeatedly issued at different times, multiple data sets corresponding to the different times, that is, data sets corresponding to different memory periods are stored as fixed data sets. Accordingly, when the display command is issued, waveforms corresponding to two or more data sets are superimposed on the screen, and the differences between the behaviors of yarns at different times can be clearly observed. Thus, it can be determined whether or not the loom is in a good operational state, and the operational state of the loom can thus be evaluated.

In the above, the sampling of the output signal from the sensor may be continuously performed while the loom is in operation, and a waveform corresponding to the latest data up to the present time may be displayed on the screen of the device in real time until the display command is issued. Alternatively, the sampling of the output signal of the sensor may be started after the issuance of the store command, and the sampled data may be successively stored over the predetermined memory period.

When the data is stored in the memory along with the crank angle at the time of sampling, it may be displayed on the screen as a waveform with the crank angle on the horizontal axis.

The sampling period and the memory period are adequately set depending on the speed of behavior of yarns to be detected by the sensor. For example, when the sensor is a warp tension sensor, the sampling period is preferably set to several tens of degrees in terms of crank angle and the memory period to several cycles of the loom. When the sensor is a weft feeler, preferably, the sampling period and the memory period are respectively set to several degrees in terms of crank angle and about 0.5 cycles or less of the loom including a period in which a weft yarn is detected at a predetermined position. The sampling period may be set in terms of either time or crank angle.

The store command may be generated either manually using a switch or the like or automatically at predetermined times in the operation of the loom, for example, at one or more of the time when a predetermined weaving time elapses, the time when the length of the woven cloth reaches a predetermined length, the time when a predetermined abnormality occurs, the time when a loom stop signal is generated, the time when the loom is activated, etc. Alternatively, the store command may also be generated periodically. With respect to the display command, it is generally sufficient to generate the command by manual operation. However, the display command may also include various options in order to, for example, select the memory period of the data to be display from multiple memory periods, designate a display range in the data corresponding to the selected memory period, etc.

### BRIEF DESCRIPTION OF THE DRAWINGS

Fig. 1 is a block diagram showing the overall structure;
Fig. 2 is an explanatory diagram showing the state of use;
Fig. 3 is an explanatory diagram showing the operation;
Fig. 4 is a conceptual diagram showing the structure of the main part;
Fig. 5 is a diagram showing a display screen (1);
Fig. 6 is a diagram showing a display screen (2);
Fig. 7 is a diagram showing a display screen (3); and
Fig. 8 is a block diagram showing the main part according to another embodiment.

### DESCRIPTION OF THE PREFERRED EMBODIMENTS

Embodiments of the present invention will be described below with reference to the accompanying drawings.

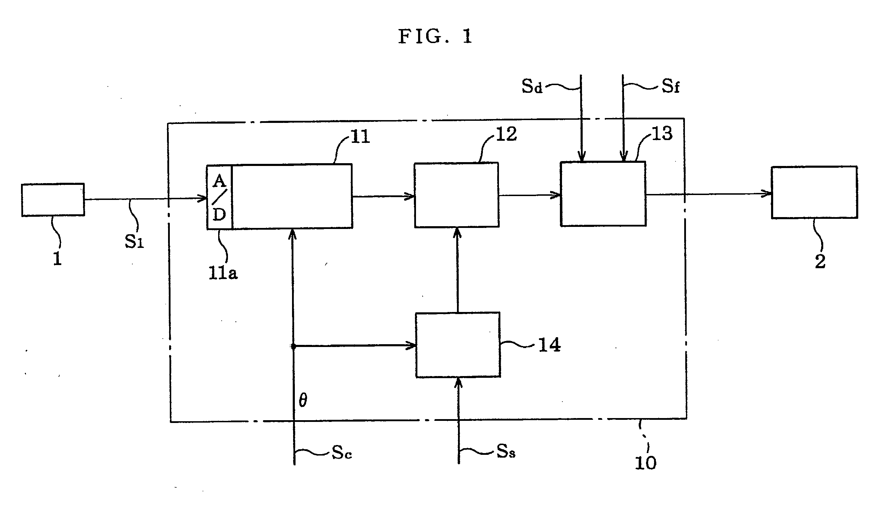

A display device 10 of a loom includes a sampling unit 11, a memory 12, and a display controller 13 as main components (Fig. 1). An A/D converter 11a is attached to the sampling unit 11.

An output signal S1 from a sensor 1 which detects the behavior of yarns is input to the sampling unit 11. An output of the sampling unit 11 is connected to an external display 2 via the memory 12 and the display controller 13. The sensor 1 is mounted on the loom to detect the behavior of warp or weft, and outputs the result of detection as the electrical output signal S1. The display 2 is a cathode-ray tube display, a liquid crystal display, a plasma display, or the like which has a display screen.

A crank angle signal Sc representing a crank angle θ of the loom is also input to the sampling unit 11. In addition, the crank angle signal Sc is also input to a memory controller 14. A store command Ss is externally input to the memory controller 14, and an output of the memory controller 14 is connected to the memory 12. A display command Sd and a shift command Sf are externally input to the display controller 13.

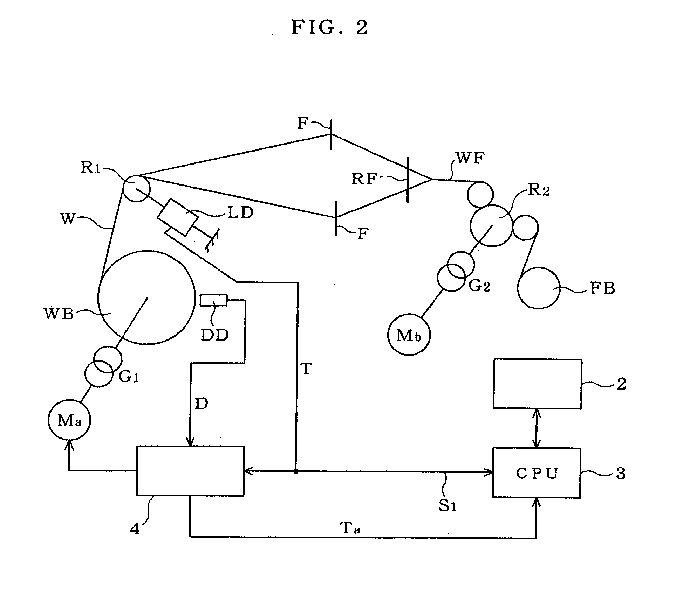

The display device 10 of the loom may be structured as software installed in a microcomputer 3 and be used for observing and evaluating the behavior of warp W from a warp tension T (Fig. 2). In the structure shown in Fig. 2, a load cell LD which detects the warp tension T for a let-off controller 4 is used as the sensor 1.

The warp W is pulled out from a warp beam WB via a tension roller R1, and a shed is formed by healds F and F. Then, a weft yarn (not shown) is woven in by being beaten with a reed RF, and a woven fabric WF is obtained accordingly. The woven fabric WF is wound around a cloth beam FB via a take-up roller R2. A let-off motor Ma is connected to the warp beam WB with a gear box G1 disposed therebetween, and a take-up motor Mb is connected to the cloth beam FB via a gear box G2 disposed therebetween.

The let-off controller 4 detects the warp tension T on the tension roller R1 with the load cell LD and a beam diameter D of the warp beam WB with a beam-diameter sensor DD, and rotates the warp beam WB while maintaining the warp tension T at a desired value by controlling the speed of the let-off motor Ma. The let-off controller 4 executes sampling control using the microcomputer 3, and a sampling period of the warp tension T and the beam diameter D is set to, for example, 20° in terms of crank angle. Data corresponding to three cycles of the loom, that is, (360/20)x3=54 data items are obtained for each of the warp tension T and the beam diameter D, and average values Ta and Da thereof are used for controlling the let-off motor Ma. The let-off controller 4 outputs the average value Ta of the warp tension T to the microcomputer 3.

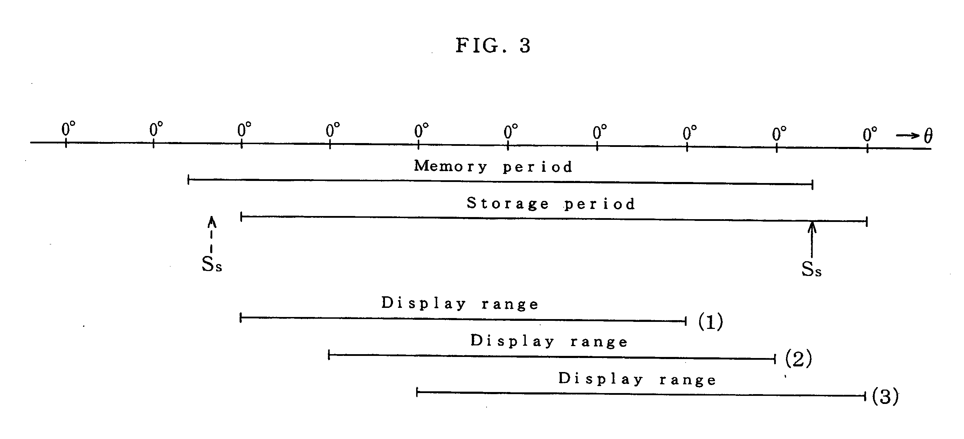

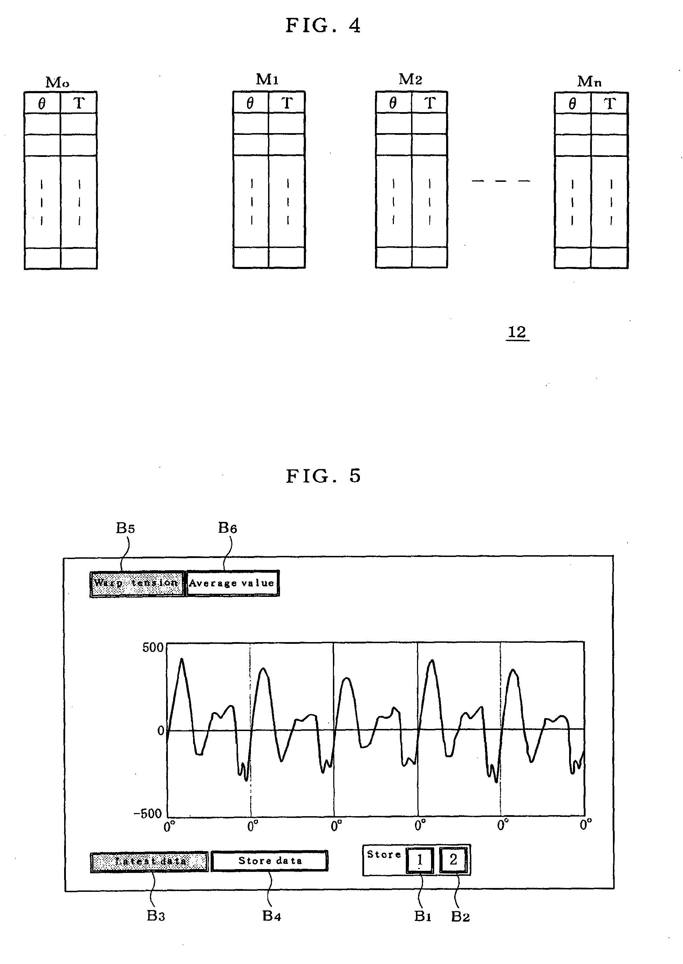

During the operation of the loom, the sampling unit 11 samples the output signal S1 from the sensor 1, that is, the warp tension T from the load cell LD, at every predetermined crank angle θ, for example, every 20°, and memorizes and updates data corresponding to a predetermined period, for example, seven cycles of the loom in a memory area Mo of the memory 12 (Figs. 3 and 4). More specifically, data corresponding to (360/20)x7=126 pairs including the crank angle θ and the warp tension T is memorized and successively updated in the memory area Mo. In Fig. 3, a memory period is shown assuming that the external store command Ss, which will be described below, is generated at the present time.

If the external store command Ss is generated (solid arrow in Fig. 3), the memory controller 14 outputs a command to the memory 12 at the time when the crank angle θ reaches 0° immediately after so that the data in the memory area Mo is transmitted and stored in another memory area M1 in the memory 12. In Fig. 3, a storage period is set the same as the memory period, that is, to the length corresponding to seven cycles, and a period shorter than one cycle is not included. The memory controller 14 may also store data corresponding to seven cycles excluding a period shorter than one cycle immediately after the generation of the store command Ss (dashed arrow in Fig. 3) in the memory area M1. Alternatively, the size of the memory area Mo in the memory controller 14 may be increased such that the memory period is longer than the storage period by at least one cycle, and when the store command Ss is generated, data within the storage period excluding a period shorter than one cycle immediately before the generation of the store command Ss may be instantaneously transmitted to the memory area M1 from the memory area Mo.

Then, when the store command Ss is generated the second time, similar to the previous time, the memory controller 14 transmits the data in the memory area Mo to another memory area M2 in the memory 12. Then, in a similar manner, the memory controller 14 transmits the data in the memory area Mo to one of memory areas Mn (n=2, 3, ...) every time the store command Ss is generated. If the store command Ss is generated more than n times, the memory controller 14 updates the oldest data in the memory areas Mn by the data currently memorized in the memory area Mo.

When the external display command Sd is not input, the display controller 13 reads out data in the memory area Mo in the memory 12 and displays a waveform corresponding to the latest data on the screen of the display 2 (Fig. 5). In Fig. 5, the horizontal axis shows the crank angle θ and the vertical axis shows the warp tension T. The screen shows, for example, a waveform of the warp tension T corresponding to five previous cycles up to the present in real time. In the screen shown in Fig. 5, two store buttons B1 and B2 are provided so that the store command Ss can be generated twice. In addition, buttons B3 and B4 are provided for switching the display contents between the data in the memory area Mo and the data in the memory area Mn, and buttons B5 and B6 are provided for switching the value to be displayed between the warp tension T and the average value Ta thereof. The waveform shown in Fig. 5 corresponds to the case in which the buttons B3 and B5 are pressed.

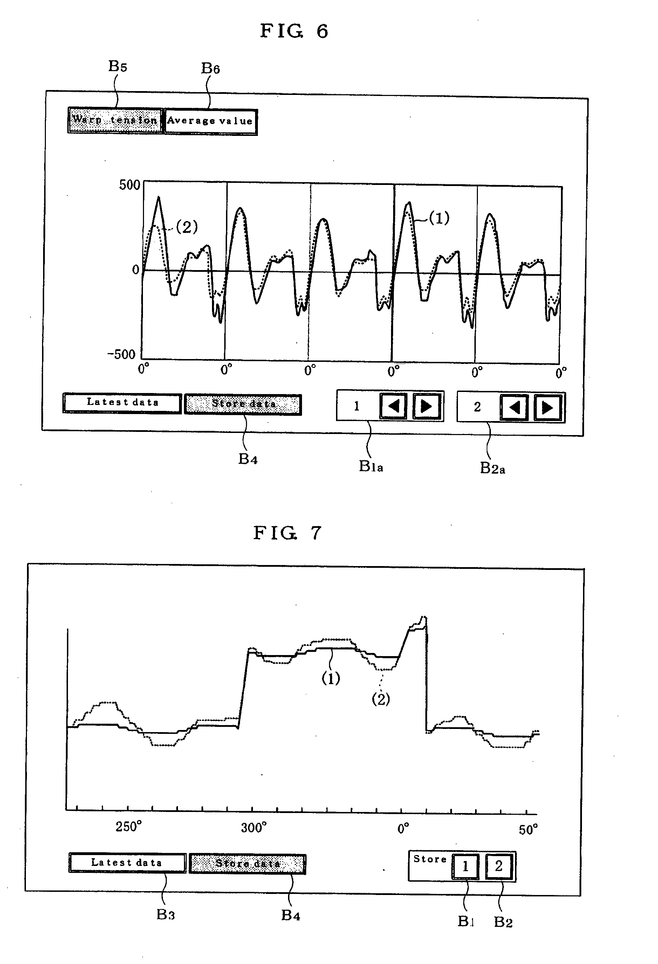

When the button B4 is pressed, the display command Sd is generated. Accordingly, the display controller 13 reads out data obtained in different memory periods stored in the memory areas M1 and M2, and superimposes them on the screen (Fig. 6). In Fig. 6, waveforms (1) and (2) correspond to data stored in memory areas M1 and M2, respectively. At this time, the store buttons B1 and B2 are changed to shift buttons B1a and B2a, respectively, on the screen. When the buttons B1a and B2a are pressed, a shift command Sf is generated. Accordingly, the display controller 13 shifts the display ranges of the waveforms (1) and (2) corresponding to the memory areas M1 and M2 in the horizontal direction individually (see (1) to (3) in Fig. 3). More specifically, the display range on the screen, which corresponds to five cycles, is set shorter than the storage period in the memory areas M1 and M2, which corresponds to seven cycles.

In Figs. 5 and 6, the vertical axes corresponding to the crank angle θ=0° are preferably color-coded for each of the waveforms (1) and (2) in accordance with the period at which the average values Ta and Da of the warp tension T and the beam diameter D, respectively, are obtained in the let-off controller 4. This is because the two waveforms (1) and (2) can be easily synchronized with each other on the screen when they are shifted individually in such a case.

In this embodiment, the store command Ss is generated by the two store buttons B1 and B2. Accordingly, only the memory areas M1 and M2 corresponding to the buttons B1 and B2, respectively, are necessary. When one of the buttons B1 and B2 is pressed multiple times, the contents in the corresponding memory area M1 or M2 are updated by the data in the memory area Mo each time the button is pressed. When the button B6 on the screen is pressed, the display controller 13 displays a graph showing the temporal variation in the warp tension up to the present time on the screen using the data of the average value Ta of the warp tension T, which is stored separately. At this time, the display range preferably covers data corresponding to several hundred cycles so that tendency of long-term variation in the warp tension T can be observed.

In the case in which a weft feeler which detects that the an inserted weft yarn has reached a predetermined position is used as the sensor 1 and the data in the memory areas M1 and M2 are superimposed on each other, a display screen shown in Fig. 7, for example, is obtained. In Fig. 7, the horizontal axis shows the crank angle θ, and the display range is shorter than one cycle. The vertical axis shows the signal level of the output signal S1 from the weft feeler. In Fig. 7, waveforms (1) and (2) respectively show a normal operational state and a state in which the variation in the signal level of the output signal S1 is abnormal.

In the above-described explanations, when the data stored in the memory areas Mn are displayed, as shown in Figs. 6 and 7, reference information including the time and date indicating the display period, the rotational speed of the loom, the shedding pattern of the healds F and F, and the kind of the inserted weft at that time may also be memorized and be additionally displayed on the screen. The reference information may also be set such that whether or not to display the reference information can be selected.

### Other Embodiments

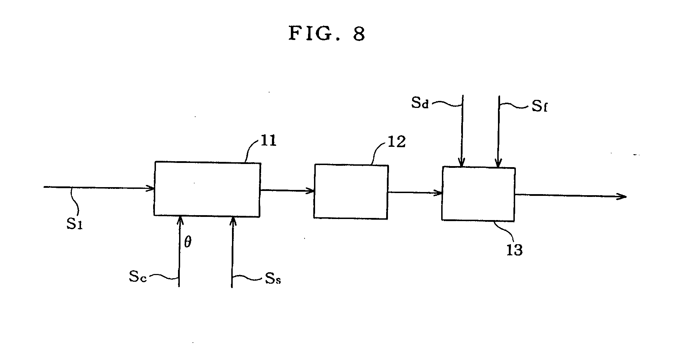

In Fig. 1, the memory controller 14 may also be omitted and both of the crank angle signal Sc and the store command Ss may be input to the sampling unit 11 (Fig. 8).

In this case, a memory 12 includes only memory areas Mn (n=1, 2, ...), and does not included the memory area Mo. The sampling unit 11 samples an output signal S1 from a sensor 1 at every predetermined crank angle θ over a predetermined memory period in response to the store command Ss, and stores the obtained data in the memory areas Mn. In response to a display command Sd, a display controller 13 superimposes waveforms corresponding to the data obtained in different memory periods and stored in the memory areas Mn of the memory 12 on a screen of the display 2. Although the latest data cannot be displayed in real time since the memory area Mo is not provided, all of the other functions can also be realized in this case.

## Claims

1. A display method for a loom including a sensor which detects the behavior of yarns, a memory (12) which memorizes an output signal (S1) from the sensor (1), and a display (2) which displays the data in the memory (12) on a screen, the display method comprising:
a step of storing a data set which is based on the output signal (S1) from the sensor (1) sampled at every predetermined period and which corresponds to a predetermined memory period in response to a store command (Ss); and
a step of displaying waveforms corresponding to a plurality of data sets obtained in different memory periods and stored in the memory (12) on the screen such that the waveforms are superimposed on each other in response to a display command (Sd).

2. The display method for the loom according to Claim 1, wherein the loom samples the output signal (S1) from the sensor (1) at every predetermined period while in operation, memorizes the sampled data in the memory (12) over the predetermined memory period, successively updates the data in the memory (12), and stores the data memorized in the memory (12) in response to the store command (Ss).

3. The display method for the loom according to Claim 1, wherein the loom samples the output signal (S1) from the sensor (1) at every predetermined period in response to the store command (Ss), successively memorizes the sampled data in the memory (12), and stores the data corresponding to the predetermined memory period in the memory (12).

4. A display device (10) for a loom including a sensor (1) which detects the behavior of yarns, a memory (12) which memorizes an output signal (S1) from the sensor (1), and a display (2) which displays the data in the memory (12) on a screen, the display device (10) comprising:
sampling means (11) for sampling the output signal (S1) from the sensor (1) at every predetermined period and causing the memory (12) to memorize the sampled data set over a predetermined memory period; and
display control means (13) which displays waveforms corresponding to a plurality of data sets obtained in different memory periods on the screen such that the waveforms are superimposed on each other in response to a display command (Sd), each of the data sets being stored in the memory (12) in response to a store command (Ss).

5. The display device (10) for the loom according to Claim 4, further comprising memory control means (14),
wherein the sampling means (11) samples the output signal (S1) from the sensor (1) at every predetermined period while the loom is in operation, memorizes the sampled data in the memory (12) over the predetermined memory period, and successively updates the data in the memory (12), and
wherein the memory control means (14) stores the data memorized in the memory (12) by the sampling means (11) and corresponding to the predetermined memory period in response to the store command (Ss).

6. The display device (10) for the loom according to Claim 4, wherein the sampling means (11) samples the output signal (S1) from the sensor (1) at every predetermined period in response to the store command (Ss), successively memorizes the sampled data in the memory (12), and stores the data corresponding to the predetermined memory period in the memory (12).

7. The display device (10) for the loom according to one of Claims 4 to 6, wherein the display control means (13) shifts display ranges of the waveforms on the screen in response to a shift command (Sf).

## Patentansprüche

1. Anzeigeverfahren für einen Webstuhl umfassend einen Sensor, der ein Verhalten von Garnen erfasst, einen Speicher (12), der ein Ausgabesignal (S1) von dem Sensor (1) speichert, und eine Anzeigevorrichtung (2), die die Daten in dem Speicher (12) auf einem Bildschirm anzeigt, wobei das Anzeigeverfahren umfasst:
einen Schritt eines Speicherns eines Datensets, das auf dem Ausgabesignal (S1) von dem Sensor (1) basiert, abgefragt zu einer jeden vorbestimmten Periode, und das mit einer vorher festgelegten Speicherperiode als Antwort auf ein Speicherkommando (Ss) korrespondiert; und
einen Schritt eines Anzeigens von Wellenformen, die zu mehreren von Datensets, die in verschiedenen Speicherperioden erhalten wurden und in dem Speicher (12) gespeichert sind auf dem Bildschirm, derart, dass die Wellenformen, als Antwort auf ein Anzeigekommando (Sd) übereinander gelagert sind.

2. Anzeigeverfahren für den Webstuhl gemäß Anspruch 1, bei dem in Betrieb der Webstuhl das Ausgabesignal (S1) von dem Sensor (1) zu jeder vorbestimmten Periode abfragt, die abgefragten Daten in dem Speicher (12) über die vorbestimmte Speicherperiode speichert, fortlaufend die Daten in dem Speicher (12) auf den neuesten Stand bringt, und die in dem Speicher (12) gespeicherten Daten als Antwort auf das Speicherkommando (Ss) speichert.

3. Anzeigeverfahren für den Webstuhl gemäß Anspruch 1, bei dem der Webstuhl das Ausgabesignal (S1) von dem Sensor (1) zu jeder vorbestimmten Periode als Antwort auf das Speicherkommando (Ss) abfragt, und fortlaufend die abgefragten Daten in dem Speicher (12) speichert, und die Daten korrespondierend zu der vorbestimmten Speicherperiode in dem Speicher (12) einspeichert.

4. Anzeigevorrichtung (10) für einen Webstuhl umfassend einen Sensor (1), der das Verhalten von Garnen erfasst, einen Speicher (12), der ein Ausgabesignal (S1) von dem Sensor (1) speichert, und eine Anzeigevorrichtung (2), die die Daten in dem Speicher (12) auf einem Bildschirm anzeigt, wobei die Anzeigevorrichtung (10) umfasst:
ein Abfragemittel (11) zum Abfragen des Ausgabesignals (S1) von dem Sensor (1) zu jeder vorbestimmten Periode und das den Speicher (12) dazu bringt, das abgefragte Datenset über eine vorbestimmte Speicherperiode zu speichern; und
ein Anzeigesteuermittel (13), das Wellenformen korrespondierend zu mehreren Datensets, die über verschiedene Speicherperioden erhalten wurden, auf dem Bildschirm anzeigt derart, dass die Wellenformen als Antwort auf ein Anzeigekommando (Sd) übereinander gelagert sind, wobei jeder der Datensets in dem Speicher (12) als Antwort auf ein Speicherkommando (Ss) eingespeichert wird.

5. Anzeigevorrichtung (10) für den Webstuhl gemäß Anspruch 4 weiter umfassend ein Speicher-Steuermittel (14),
wobei das Abfragemittel (11) das Ausgabesignal (S 1) von dem Sensor (1) zu jeder vorbestimmte Periode abfragt, während der Webstuhl sich im Betrieb befindet, die abgefragten Daten in dem Speicher (12) über die vorbestimmte Speicherperiode speichert, und fortlaufend die Daten in dem Speicher (12) auf den neuesten Stand bringt, und
wobei das Speicher-Steuermittel (14) die Daten, die in dem Speicher (12) über das Abfragemittel (11) gespeichert sind und die zu der vorher festgelegten Speicherperiode als Antwort auf das Speicherkommando (Ss) korrespondieren, speichert.

6. Anzeigevorrichtung (10) für den Webstuhl gemäß Anspruch 4, bei dem das Abfragemittel (11) das Ausgabesignal (S1) von dem Sensor (1) zu jeder vorbestimmten Periode als Antwort auf das Speicherkommando (Ss) speichert, und fortlaufend die abgefragten Daten in dem Speicher (12) speichert, und die Daten korrespondierend zu der vorbestimmten Speicherperiode in dem Speicher (12) einspeichert.

7. Anzeigevorrichtung (10) für den Webstuhl gemäß einem der Ansprüche 4 bis 6, bei dem das Anzeige-Steuermittel (13) Anzeigebereiche der Wellenformen auf dem Bildschirm als Antwort auf ein Änderungskommando (Sf) verändert.

## Revendications

1. Procédé d'affichage pour un métier à tisser comprenant un capteur qui détecte le comportement de fils, une mémoire (12) qui mémorise un signal de sortie (S1) provenant du capteur (1), et un afficheur (2) qui affiche les données se trouvant dans la mémoire (12) sur un écran, le procédé d'affichage comprenant :
une étape consistant à enregistrer un ensemble de données qui est basé sur le signal de sortie (S1) provenant du capteur (1) échantillonné à chaque période prédéterminée et qui correspond à une période de mémoire prédéterminée en réponse à une commande d'enregistrement (Ss) ; et
une étape consistant à afficher des formes d'onde correspondant à une pluralité d'ensembles de données obtenus au cours de différentes périodes de mémoire et enregistrés dans la mémoire (12) sur l'écran de façon à ce que les formes d'onde soient superposées l'une sur l'autre en réponse à une commande d'affichage (Sd).

2. Procédé d'affichage pour le métier à tisser selon la revendication 1, dans lequel le métier échantillonne le signal de sortie (S1) provenant du capteur (1) à chaque période prédéterminée pendant le fonctionnement, mémorise les données échantillonnées dans la mémoire (12) au cours de la période de mémoire prédéterminée, actualise successivement les données dans la mémoire (12), et enregistre les données mémorisées dans la mémoire (12) en réponse à la commande d'enregistrement (Ss).

3. Procédé d'affichage pour le métier à tisser selon la revendication 1, dans lequel le métier échantillonne le signal de sortie (S1) provenant du capteur (1) à chaque période prédéterminée en réponse à la commande d'enregistrement (Ss), mémorise successivement les données échantillonnées dans la mémoire (12) et enregistre les données correspondant à la période de mémoire prédéterminée dans la mémoire (12).

4. Dispositif d'affichage (10) pour un métier à tisser comprenant un capteur (1) qui détecte le comportement de fils, une mémoire (12) qui mémorise un signal de sortie (S1) provenant du capteur (1), et un afficheur (2) qui affiche les données se trouvant dans la mémoire (12) sur un écran, le dispositif d'affichage (10) comprenant :
un moyen d'échantillonnage (11) pour échantillonner le signal de sortie (S1) provenant du capteur (1) à chaque période prédéterminée et faire en sorte que la mémoire (12) mémorise l'ensemble de données échantillonné au cours d'une période de mémoire prédéterminée ; et
un moyen de contrôle de l'affichage (13) qui affiche des formes d'onde correspondant à une pluralité d'ensembles de données obtenus au cours de différentes périodes de mémoire sur l'écran de telle sorte que les formes d'onde sont superposées l'une sur l'autre en réponse à une commande d'affichage (Sd), chacun des ensembles de données étant enregistré dans la mémoire (12) en réponse à une commande d'enregistrement (Ss).

5. Dispositif d'affichage (10) pour le métier à tisser selon la revendication 4, comprenant en outre des moyens de contrôle de la mémoire (14),
dans lequel le moyen d'échantillonnage (11) échantillonne le signal de sortie (S1) provenant du capteur (1) à chaque période prédéterminée pendant que le métier fonctionne, mémorise les données échantillonnées dans la mémoire (12) au cours de la période de mémoire prédéterminée, et actualise successivement les données dans la mémoire (12), et dans lequel le moyen de contrôle de la mémoire (14) enregistre les données mémorisées dans la mémoire (12) par l'unité d'échantillonnage (11) et correspondant à la période de mémoire prédéterminée en réponse à la commande d'enregistrement (Ss).

6. Dispositif d'affichage (10) pour le métier à tisser selon la revendication 4, dans lequel le moyen d'échantillonnage (11) échantillonne le signal de sortie (S1) provenant du capteur (1) à chaque période prédéterminée en réponse à la commande d'enregistrement (Ss), mémorise successivement les données échantillonnées dans la mémoire (12) et enregistre les données correspondant à la période de mémoire prédéterminée dans la mémoire (12).

7. Dispositif d'affichage (10) pour le métier à tisser selon l'une des revendications 4 à 6, dans lequel le moyen de contrôle de l'affichage (13) déplace les marges d'affichage des formes d'onde sur l'écran en réponse à une commande de déplacement (Sf).
